Europäisches Patentamt

European Patent Office

Office européen des brevets

(19)

(11) Veröffentlichungsnummer: **0 243 914**
**A2**

## (12) EUROPÄISCHE PATENTANMELDUNG

(21) Anmeldenummer: 87106052.1

(22) Anmeldetag: 25.04.87

(51) Int. Cl.³: **C 12 M 3/00**
**C 12 M 1/12**

(30) Priorität: 30.04.86 DE 3614712

(43) Veröffentlichungstag der Anmeldung:
04.11.87 Patentblatt 87/45

(84) Benannte Vertragsstaaten:
CH DE FR GB LI NL SE

(71) Anmelder: Diessel GmbH & Co.
Steven 1
D-3200 Hildesheim-Bavenstedt(DE)

(84) Benannte Vertragsstaaten:
CH DE FR GB LI NL SE

(71) Anmelder: Gesellschaft für Biotechnologische
Forschung mbH (GBF)
Mascheroder Weg 1
D-3300 Braunschweig-Stöckheim(DE)

(84) Benannte Vertragsstaaten:
CH DE FR GB LI NL SE

(72) Erfinder: Lehmann, Jürgen, Dr.-Ing.
Bolkenhain 2 d
D-3300 Braunschweig(DE)

(74) Vertreter: Wehser, Wulf, Dipl.-Ing. et al,
Roscherstrasse 12
D-3000 Hannover 1(DE)

(54) **Vorrichtung zur Kultivierung von Zellkulturen.**

(57) Eine Vorrichtung zur Kultivierung von Zellkulturen, bei welcher die Kulturen sich innerhalb wenigstens eines flüssigen Nährmediums in einem geschlossenen Behälter befinden, wobei das Nährmedium oder die Nährmedien dem Behälter zuführbar und aus diesem über zwischengeschaltete Filter entfernbar sind, soll so ausgebildet werden, daß Filter Verwendung finden, die eine wesentlich größere Standzeit aufweisen und bei denen ein Verstopfen ausgeschlossen ist.

Hierzu ist erfindungsgemäß vorgesehen, daß ein flexibles Filtergewebe abdichtend, aber zugleich locker beweglich aufgehängt ist, wobei das Filter innerhalb des Nährmediums bewegbar ist.

EP 0 243 914 A2

Croydon Printing Company Ltd.

**'EHSER & FLEUCHAUS**
PATENTANWÄLTE
European Patent Attorneys

DIPL.-ING. WULF WEHSER
3000 Hannover 1
Roscherstraße 12
☎ 0511-341449
Privat: 05143-6509

DIPL.-ING. LEO FLEUCHAUS
8000 München 71
☎ 089-792800

Hannover, 1. April 1986

**0243914**

-1-

Diessel GmbH & Co., Steven 1, 3200 Hildesheim-Bavenstedt

GBF Gesellschaft für Biotechnologische Forschung mbH,
Mascheroder Weg 1, 3300 Braunschweig

D 150 - Ws/Me

## Vorrichtung zur Kultivierung von Zellkulturen

Die Erfindung betrifft eine Vorrichtung zur Kultivierung von
Zellkulturen, bei welcher die Kulturen sich innerhalb wenigstens
eines flüssigen Nährmediums in einem geschlossenen Behälter
befinden, wobei das Nährmedium oder die Nährmedien dem Behälter
zuführbar und aus diesem über zwischengeschaltete Filter entfernbar sind.

Bei bekannten Vorrichtungen dieser Art besteht die Notwendigkeit, von Zeit zu Zeit die Nährflüssigkeit innerhalb des
Behälters auszutauschen und in Zusammenhang damit das abzuspaltende Produkt (biologische Moleküle, Proteine od. dgl./
auszufördern. In solchen Vorrichtungen sind im allgemeinen
kugelförmige Kleinstkörper enthalten, sogenannte Microcarriers
(ca. 50 µ Durchmesser), auf denen die Zellen der Kulturen
wachsen.

Es besteht nun die Schwierigkeit, den Flüssigkeitsaustausch so vorzunehmen, daß die Microcarriers innerhalb der Flüssigkeit während des Austauschvorganges verbleiben.

Zu diesem Zweck ist es bereits bekannt geworden, die Flüssigkeitsabfuhr über sogenannte Spinfilter vorzunehmen, die drehbar innerhalb der Flüssigkeit angeordnet sind. Die Drehbarkeit der Filter schließt aber nicht aus, daß sich die Zellenträger (Microcarriers) an der Oberfläche des Filters anlagern und damit das Filter verstopfen, so daß ohne Austausch des Filters der Abfuhrvorgang nicht weitergehen kann.

Besonders schwerwiegende Nachteile bestehen hierbei darin, daß aufgrund solcher Unterbrechungen die Sterilität nicht mehr gewährleistet ist und daß insbesondere der eingeleitete Prozeß nicht mehr fortgesetzt werden kann, so daß das Gesamtverfahren wiederholt werden muß.

Der Erfindung liegt demgemäß die Aufgabe zugrunde, eine Vorrichtung der eingangs genannten Art so auszubilden, daß Filter Verwendung finden, die eine wesentlich größere Standzeit aufweisen und bei denen ein Verstopfen ausgeschlossen ist.

Diese Aufgabe wird erfindungsgemäß dadurch gelöst, daß das Filter wenigstens eine Durchtrittsfläche aufweist, in welcher ein flexibles Filtergewebe abdichtend, aber zugleich locker beweglich aufgehängt ist, wobei das Filter innerhalb des Nährmediums bewegbar ist.

Mit dieser Anordnung wird erreicht, daß durch die Bewegung des Filters innerhalb der Flüssigkeit im Behälter das locker aufgehängte Filtergewebe in Bewegung gerät, also gleichsam flattert, wobei diese Flatterbewegung zu einem Abfallen der gegen das Filter geführten Microcarriers führt und/oder das Anhaften der Microcarriers von vornherein verhindert.

Für die Wirkung der erfindungsgemäßen Anordnung kann es genügen, wenn eine ebene Filterfläche in Form eines Rahmens vorgesehen ist, dessen Öffnung von dem Filtergewebe bedeckt ist, welches abdichtend an den Rahmen angeschlossen ist, und dessen der Abfuhrleitung zugewandte Rückseite kastenförmig ausgebildet ist. Der an den Rahmen anschließende Kasten kann dann direkt an die Abfuhrleitung unter Zwischenschaltung eines flexiblen Schlauchstückes angeschlossen sein.

Besonders vorteilhaft ist es jedoch, wenn das Filter aus einem Filtersack besteht, der nach oben offen ist und dessen offenes Ende durch eine Kopfplatte verschlossen ist, an deren
Umfang das Filtergewebe abdichtend angeschlossen ist.

Mit einer solchen Ausbildung wird eine besonders große
Filterfläche erreicht und außerdem ein hinreichendes
Flattern innerhalb der Flüssigkeit sichergestellt. Dieses
Flattern ist insbesondere dann gewährleistet, wenn die
Kopfplatte und damit das Filter eine hin- und hergehende
Bewegung ausführen,insbesondere eine Taumelbewegung.

Die Kopfplatte kann in besonders vorteilhafter Weise mit der
Abfuhrleitung oder dem flexiblen Zwischenstück in Verbindung
stehen.

Vorteilhaft ist es weiter, wenn die Kopfplatte ein nach unten
weisendes Drahtgestell trägt, über welches im Abstand zu
diesem der Filtersack gezogen ist. Ein solches Drahtgestell
hat einerseits die Wirkung, den Filtersack über seine gesamte
Höhe ausgespreizt zu halten und führt andererseits dazu, daß
das Filtergewebe bei der Bewegung des Filters durch das Drahtgestell gewalkt wird, was zu einem Lösen etwa anhaftender
Microcarriers beiträgt.

Besonders vorteilhaft ist es, wenn das Filtergewebe ein
hydrophobes feinmaschiges Gewebe, beispielsweise ein
Nylongewebe oder ein anderes Kunststoffgewebe, ist.

Die erfindungsgemäße Anordnung ist daher besonders für
eine Vorrichtung geeignet, die der blasenfreien Begasung
der Zellkulturen dient.

Wie oben ausgeführt, ist eine Hin- und Herbewegung oder
eine Taumelbewegung des Filters besonders vorteilhaft,
wobei das Filter in diesem Fall an einen die Membranen
für die Begasung halternden Membrankorb angeschlossen sein
kann.

Es ist aber auch möglich, das Filter rotieren zu lassen,da
durch das Drahtgestell und die Anlage des Filtergewebes an
diesem sich Wirbel bilden, die zu einem Flattern des Filtergewebes führen.

Die Maschenweite des Filtergewebes soll so gewählt werden,
daß einerseits die Microcarriers zurückgehalten, daß andererseits aber die freien Zellen abgefördert werden.

Die Erfindung wird im folgenden anhand von Ausführungsbeispielen in der Zeichnung näher erläutert.

Fig. 1          zeigt in schemtischer Darstellung
                eine Ausführungsform der erfindungsgemäßen Vorrichtung.

Fig. 2                zeigt in schematischer Darstellung eine Ausführungsform des erfindungsgemäß ausgebildeten Filters.

Fig. 3                zeigt in schematischer Darstellung eine andere Ausführungsform des Filters.

Fig. 4                ist der Schnitt IV-IV nach Fig. 1.

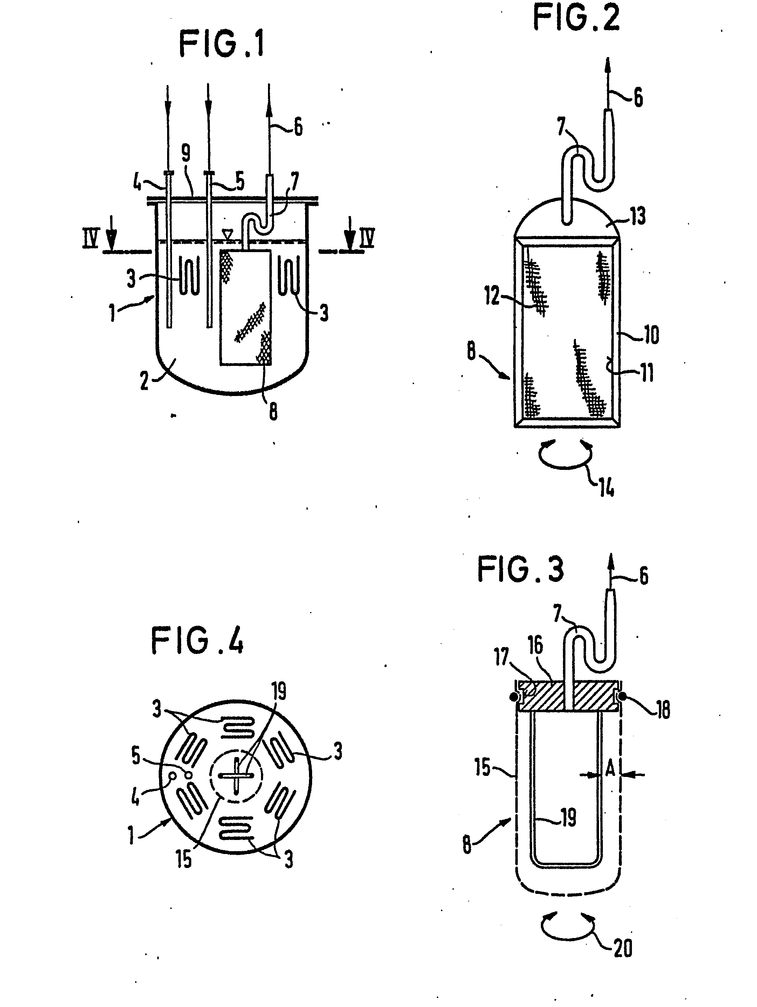

Gemäß Fig. 1 ist ein Behälter 1 vorgesehen, in welchem sich in
einer Nährmittelflüssigkeit 2 Zellkulturen befinden, die kultiviert werden sollen. Hierzu kann beispielsweise über Membranschläuche 3 die Flüssigkeit 2 begast werden.

Da die Nährmittelflüssigkeit 2 im Behälter 1 von Zeit zu Zeit
ersetzt werden muß, sind in die Flüssigkeit 2 eintauchende
Rohre 4 und 5 vorgesehen, über welche Nährmedien der Flüssigkeit 2 im Behälter 1 zugeführt werden können. Dieselbe Flüssigkeitsmenge, die zugeführt wird, muß auch wieder abgeführt
werden, wozu eine Abfuhrleitung 6 vorgesehen ist, die über
ein schwanenhalsförmiges elastisches Zwischenstück 7 mit einem
Filter 8 in Verbindung steht, welches gänzlich in die Flüssigkeit 2 eintaucht. Das Filter 8 ist so an die Abfuhrleitung 6
bzw. das Zwischenstück 7 angeschlossen, daß die gesamte
abzuführende Flüssigkeitsmenge das Filter 8 passieren muß.

Der Behälter 1 ist durch einen Deckel 9 verschlossen.

In der Flüssigkeit 2 sind Microcarriers enthalten, auf denen die Zellen der Kulturen wachsen und die durch das Filter 8 zurückgehalten werden sollen.

Das Filter 8 kann innerhalb der Flüssigkeit 2 hin- und herbewegt und/oder gedreht werden.

Fig. 2 zeigt eine einfache Ausführungsform des erfindungsgemäßen Filters. Diese besteht aus einem Rahmen 10, dessen Öffnung 11 von dem Filtergewebe 12 bedeckt ist, welches abdichtend an den Rahmen 10 angeschlossen ist. Das Filtergewebe 12 ist nicht straff über die Fläche der Öffnung 11 gespannt, sondern locker beweglich aufgehängt, um während der Bewegung des Filters flattern zu können, so daß das Anhaften der Microcarriers verhindert wird bzw. etwa anhaftende Microcarriers wieder gelöst werden.

An den Rahmen 10 schließt abfuhrseitig ein Kasten 13 an, der mit der Abfuhrleitung 6 bzw. dem Zwischenstück 7 in Verbindung steht, so daß die gesamte abzuführende Flüssigkeit die Filterfläche passieren muß.

Wie mittels des Pfeiles 14 angedeutet, kann das Filter nach Fig. 2 innerhalb der Flüssigkeit 2 hin- und hergedreht werden.

Andere Bewegungen sind möglich.

Bei dem Filtergewebe handelt es sich um ein hydrophobes feinmaschiges Gewebe, beispielsweise um ein Nylongewebe oder ein anderes Kunststoffgewebe. Das Filtergewebe hat eine Maschenweite, die so groß ist, daß einerseits die Microcarriers zurückgehalten und andererseits die freien Zellen abgefördert werden.

Fig. 3 zeigt eine andere Ausführungsform der Filterausbildung. Bei dieser Ausführungsform besteht das Filter 8 aus einem Filtersack 15, der nach oben offen ist und dessen offenes Ende durch eine Kopfplatte 16 verschlossen ist, an deren Umfang das Filtergewebe abdichtend angeschlossen ist. Hierzu ist bei der Ausführungsform nach Fig. 3 eine umlaufende Nut 17 vorgesehen, in welche das Filtergewebe im Randbereich des Filtersackes eingedrückt und dort durch einen Spann- oder Klemmring 18 festgehalten ist.

Die Kopfplatte 16 ist direkt mit der Abfuhrleitung 6 bzw. dem flexiblen Zwischenstück 7 verbunden.

Die Kopfplatte 16 trägt ein nach unten weisendes Drahtgestell 19, über welches im Abstand A zu diesem der Filtersack gezogen ist. Das Drahtgestell 19 kann als einfacher Bügel ausgebildet sein, es ist jedoch möglich, mehrere Bügel ineinandergreifend anzuordnen, so daß ein der Zylinderform angenähertes Gestänge entsteht (vgl. Fig. 4).

Das Filter 8 gemäß der Ausführungsform nach Fig. 3 kann ebenfalls ein hin- und hergehende Bewegung entsprechend dem Pfeil 20 ausführen. Die Bewegung kann auch eine Taumelbewegung sein, und schließlich kann das Filter ebenso wie das Filter der Ausführungsform nach Fig. 2 rotieren.

Insbesondere ist es möglich, Filter beider Ausführungsformen mit einem beweglichen Membrankorb zu verbinden, der die Membranen 3 für die blasenfreie Begasung der Zellkulturen haltert und ebenfalls einer Bewegung innerhalb der Flüssigkeit 2 unterworfen ist.

Fig. 4 ist der Schnitt IV-IV nach Fig. 1, wobei die Ausbildung des Filters gemäß Fig. 3 Verwendung findet. Wie aus Fig. 4 hervorgeht, können innerhalb des Filtersackes 15 wenigstens zwei Drahtbügel 19 kreuzweise angeordnet sein und damit ein den Filtersack 15 aufspreizendes Gestell bilden. Das so ausgebildete Filter kann zentral innerhalb des Behälters 1 angeordnet sein, wobei die Membranen 3, die an einem hier nicht dargestellten Membrankorb gehaltert sind, das Filter umgeben.

**0243914**

**WEHSER & FLEUCHAUS**

PATENTANWÄLTE

European Patent Attorneys

DIPL.-ING. WULF WEHSER
3000 Hannover 1
Roscherstraße 12
☎ 0511-341449
Privat: 05143-6509

DIPL.-ING. LEO FLEUCHAUS
8000 München 71
☎ 089-792800

Hannover, 1. April 1986

Diessel GmbH & Co., Steven 1, 3200 Hildesheim-Bavenstedt

GBF Gesellschaft für Biotechnologische Forschung mbH,
Mascheroder Weg 1, 3300 Braunschweig

D 150 - Ws/Me

## A n s p r ü c h e

1. Vorrichtung zur Kultivierung von Zellkulturen, bei welcher die Kulturen sich innerhalb wenigstens eines flüssigen Nährmediums in einem geschlossenen Behälter befinden, wobei das Nährmedium oder die Nährmedien dem Behälter zuführbar und aus diesem über zwischengeschaltete Filter entfernbar sind, dadurch gekennzeichnet, daß das Filter (8) wenigstens eine Durchtrittsfläche aufweist, in welcher ein flexibles Filtergewebe (12,15) abdichtend, aber zugleich locker beweglich aufgehängt ist, wobei das Filter (8) innerhalb des Nährmediums (2) bewegbar ist.

2. Vorrichtung nach Anspruch 1, dadurch gekennzeichnet, daß eine ebene Filterfläche (12) in Form eines Rahmens (10)

- 2 -

vorgesehen ist, dessen Öffnung (11) von dem Filtergewebe (12) bedeckt ist, welches abdichtend an den
Rahmen (10) angeschlossen ist.

3. Vorrichtung nach Anspruch 2, dadurch gekennzeichnet, daß
die der Abfuhrleitung (6,7) zugewandte Rückseite des Rahmens (10)
kastenförmig ausgebildet ist, wobei der Kasten (13) an die
Abfuhrleitung (6,7) angeschlossen ist.

4. Vorrichtung nach Anspruch 1, dadurch gekennzeichnet, daß
das Filter (8) aus einem Filtersack (15) besteht, der nach
oben offen ist und dessen offenes Ende durch eine Kopfplatte (16) verschlossen ist, an deren Umfang das Filtergewebe abdichtend angeschlossen ist.

5. Vorrichtung nach Anspruch 4, dadurch gekennzeichnet, daß
die Kopfplatte (16) und damit das Filter (8) eine hin-
und hergehende Bewegung innerhalb der Flüssigkeit (2)
ausführen.

6. Vorrichtung nach Anspruch 4, dadurch gekennzeichnet, daß
die Kopfplatte (16) und damit das Filter (8) innerhalb der
Flüssigkeit (2) eine Taumelbewegung ausführen.

7. Vorrichtung nach einem der Ansprüche 4 bis 6, dadurch gekennzeichnet, daß die Kopfplatte (16) mit der Abfuhrleitung (6) oder einem flexiblen Zwischenstück (7) in Verbindung steht.

8. Vorrichtung nach einem der Ansprüch 4 bis 7, dadurch gekennzeichnet, daß die Kopfplatte (16) ein nach unten weisendes Drahtgestell (19) trägt, über welches im Abstand (A) zu diesem der Filtersack (15) gezogen ist.

9. Vorrichtung nach einem der vorhergehenden Ansprüche, dadurch gekennzeichnet, daß das Filtergewebe ein hydrophobes feinmaschiges Gewebe ist.

10. Vorrichtung nach Anspruch 9, dadurch gekennzeichnet, daß das Filtergewebe ein Nylongewebe oder ein anderes Kunststoffgewebe ist.

11. Anwendung der Vorrichtung nach einem der vorhergehenden Ansprüche auf eine Vorrichtung, die der blasenfreien Begasung der Zellkulturen dient.

12. Vorrichtung nach Anspruch 11, dadurch gekennzeichnet, daß das Filter (8) an einen die Membranen (3) für die Begasung halternden Membrankorb angeschlossen ist.

13. Vorrichtung nach einem der Ansprüche 4 bis 11, dadurch gekennzeichnet, daß das Filter (8) eine Drehbewegung um eine senkrechte Achse ausführt.

14. Vorrichtung nach einem der vorhergehenden Ansprüche, dadurch gekennzeichnet, daß das Filtergewebe eine Maschenweite hat, durch welche einerseits die Microcarriers zurückgehalten, andererseits aber die freien Zellen abgefördert werden.

0243914

FIG.1

FIG.2

FIG.4

FIG.3